# EUROPEAN PATENT APPLICATION

(11) **EP 4 335 396 A1**
(43) Date of publication of application: **13.03.2024**
(21) Application number: 21939917.7
(22) Date of filing: 02.12.2021
(51) Int. Cl.: A61B 18/14, A61B 18/12, A61B 90/00, A61B 18/00

(54) **ELECTRODE DEVICE COMPRISING HIGH-FREQUENCY ELECTRODE AND MULTI-FUNCTIONAL ELECTRODE**

(30) Priority: 07.05.2021 KR 20210059484
(71) Applicant: Starmed Co., Ltd., Gyeonggi-do 10442 (KR)
(72) Inventor: SHIN, Kyung Hoon, Gimpo-si Gyeonggi-do 10111 (KR); LEE, Jun Hyok, Gimpo-si Gyeonggi-do 10084 (KR); KIM, Dong Un, Gimpo-si Gyeonggi-do 10068 (KR); HONG, Sang Pyo, Paju-si Gyeonggi-do 10869 (KR); LEE, Jeong Min, Seoul 06549 (KR)
(74) Representative: Stöckeler, Ferdinand
(86) International application number: PCT/KR2021/018125
(87) International publication number: WO 2022/234914

(57) **Abstract**

An embodiment of this disclosure provides an electrode device includes a plurality of high-frequency electrodes for cauterization of a lesion tissue; a multi-functional electrode for injecting a fluid or measuring a temperature of a surrounding tissue; a temperature sensor provided inside the multi-functional electrode; and a main body which has an electrode line for supplying a high frequency to each of the plurality of high-frequency electrodes, a cooling line for circulating and supplying cooling water to the inside of each of the plurality of high-frequency electrodes, a fluid line for supplying a fluid to the inside of the multi-functional electrode, and a temperature sensor line connected to the temperature sensor, and which is coupled to the plurality of high-frequency electrodes and the multi-functional electrode via flexible tubes, respectively.

## Description

### [Technical Field]

The present invention relates to an electrode device, and more specifically, to an electrode device including a high-frequency electrode for cauterization of a lesion tissue and a multi-functional electrode for fluid injection and temperature measurement of a surrounding tissue.

### [Background Art]

Generally, when cancer tissue occurs in a body organ, it is treated with nonoperative methods or surgical operations.

Surgical operations have problems such as usually leaving large scars due to their large regions of required excision of the body at the lesion site, and requiring a long recovery period.

For this reason, nonoperative methods such as transarterial chemoembolization (TACE), percutaneous ethanol injection, systemic chemotherapy, and local thermotherapy have recently been known, among which local thermotherapy is the most effective.

Examples of this local thermotherapy include radiofrequency ablation, microwave cauterization, and laser cauterization, and the like, among which thermal therapy using high frequency is the most effective.

The above-described high-frequency thermal therapy is a method of treating cancer tissue occurred in a body organ, for example, the liver, by cauterizing only the cancer tissue with high-frequency heat and causing necrosis without excision.

A conventional electrode device for high-frequency thermal therapy is disclosed in Korean Unexamined Patent Application Publication No. 2004-0074541. In this electrode device for high-frequency thermal therapy, an electrode needle (6) is assembled to the front portion of a gripping body (5), and both an electrode line (4) that supplies a high frequency to the electrode needle (6), and a cooling line (3) that circulates and supplies cooling water to the inside of the electrode needle (6) to prevent the carbonization phenomenon of the electrode needle are installed on the rear portion of the gripping body (5). The electrode needle (6) usually has a current-carrying portion (6a) in a portion of the leading end, and an insulating portion (6b) in the remaining portion.

When taking a look at a medical procedure below, the electrode line (4) is connected to a high frequency generator (not shown), and another electrode line of the high frequency generator is connected to the pad (2) which is brought into contact with a human body. In addition, a coolant pump (not shown) is connected to the cooling line (3) to circulate and supply the coolant to the inside of the cooling needle (6).

In this prepared state, the electrode needle (6) is inserted to penetrate a lesion site (100) such as cancer tissue of a body organ, and then the high frequency is supplied to the electrode needle (6) by the high frequency generator, so that the high frequency is supplied to the current carrying portion (6a) excluding the insulating portion (6b) of the electrode needle, causing the cauterization and necrotization of the lesion site (100) by the high-frequency heat.

To the gripping body (5) of the conventional electrode device, one or more electrode needles are coupled, so that when the area of the lesion site is small, one electrode needle is used, while when the area of the lesion site is large, multiple electrode needles are used. However, there was a problem in that other electrode needles had to be used additionally when the lesion sites were scattered.

Further, in order to adjust the length of the current-carrying portion (6a) of the electrode needle (6) or inject a fluid such as a medicament according to the size of the lesion site, a guide port having a separate guide tube must be used, which leads to problems such as the complicated structure and the incapability of adjusting fluid injection position.

### [Summary of Invention]

### [Technical Problem]

The object of the present invention is to provide an electrode device including a high-frequency electrode for cauterization of a lesion tissue and a multi-functional electrode for fluid injection and temperature measurement of a surrounding tissue.

Technical objects, which the present disclosure is to accomplish, are not limited to the aforementioned ones, and other technical objects not mentioned above may be clearly appreciated from the following detailed description by a person having ordinary skill in the art to which the present disclosure belongs.

### [Solution to Problem]

In order to solve the above-mentioned problems, an aspect of the present disclosure provides an electrode device including a plurality of high-frequency electrodes for cauterization of a lesion tissue; a multi-functional electrode for injecting a fluid or measuring a temperature of a surrounding tissue; a temperature sensor provided inside the multi-functional electrode; and a main body which has an electrode line for supplying a high frequency to each of the plurality of high-frequency electrodes, a cooling line for circulating and supplying cooling water to an inside of each of the plurality of high-frequency electrodes, a fluid line for supplying a fluid to an inside of the multi-functional electrode, and a temperature sensor line connected to the temperature sensor, and which is coupled to the plurality of high-frequency electrodes and the multi-functional electrode via flexible tubes, respectively.

According to an embodiment, when the multi-functional electrode measures the temperature of the surrounding tissue with the temperature sensor, no fluid may be supplied through the fluid line.

According to an embodiment, each of the high-frequency electrodes may include a first gripping body coupled to the main body through a flexible tube; and a first electrode needle coupled to a front portion of the first gripping body, and the multi-functional electrode may include a second gripping body coupled to the main body through a flexible tube; and a second electrode needle coupled to a front portion of the second gripping body.

According to an embodiment, at an end portion of the second electrode needle, a plurality of injection holes through which a fluid is injected may be formed.

According to an embodiment, the temperature sensor may be disposed at an end portion of the second electrode needle.

According to an embodiment, the multi-functional electrode may further include a second movable part movably installed on the second gripping body; and a second insulating tube coupled to the second movable part and arranged to surround the second electrode needle.

According to an embodiment, when the fluid is supplied to the multi-functional electrode through the fluid line, the second insulating tube may not cover the plurality of injection holes, and when the multi-functional electrode measures a temperature with the temperature sensor, the second insulating tube may cover the plurality of injection holes.

According to an embodiment, each of the high-frequency electrodes may further include a first movable part movably installed on the first gripping body, and a first insulating tube coupled to the first movable part and arranged to surround the first electrode needle.

According to an embodiment, the first gripping body and the second gripping body may be integrally combined to or separable from each other.

### [Advantageous Effects of Invention]

According to the present disclosure, since the plurality of high-frequency electrodes and the multi-functional electrode are coupled to the main body through the flexible tubes, not only the cauterization position of the high-frequency electrode can be easily adjusted, but also the fluid injection position of the multi-functional electrode can also be easily adjusted.

In addition, the multi-functional electrode can measure the actual temperature of the surrounding tissue when the fluid is not supplied, so if there is an organ around the lesion tissue that should not be damaged, the damage can be prevented by measuring the actual temperature of the surrounding tissue.

Also, by adjusting the position of the insulating tube of the high-frequency electrode through the movable part installed movably on the gripping body, the exposed length of the electrode needle of the high-frequency electrode can be easily adjusted according to the size of the lesion tissue.

Further, by adjusting the position of the insulating tube of the multi-functional electrode with the movable part, whether the injection hole of the multi-functional electrode is exposed is controlled, so that when the temperature is measured with the temperature sensor inside the multi-functional electrode, the insulating tube can cover the injection hole to prevent tissue from entering and blocking the injection hole.

Furthermore, the multiple electrode needles can be used all at one time or individually by combining or separating the multiple grip bodies depending on the area of the lesion tissue and scattered locations of the lesion tissues, thereby increasing versatility in use.

The effects of the present disclosure are not limited to the above-described ones, but should be understood as including all effects that can be inferred from the configuration provided by the detailed description or claims of the present application.

### [Brief Description of Drawings]

FIG. 1 is a perspective view showing a structure of an electrode device according to an embodiment of the present invention.
FIG. 2 is a partially enlarged view of FIG. 1 with a part of a flexible tube removed.
FIG. 3 is a cross-sectional view showing a front portion of a high-frequency electrode.
FIG. 4 is a cross-sectional view showing a front portion of a multi-functional electrode.
FIG. 5 is a front view showing a rear portion of the multi-functional electrode with the flexible tube and a cover part of the gripping body removed.
FIG. 6 is a diagram schematically showing the disposition of the electrode needle during a medical procedure using the electrode device of FIG. 1.

### [Best Mode]

Hereinafter, a preferred embodiment of an electrode device of the present invention will be described with reference to the accompanying drawings.

In addition, the terms described below are ones defined in view of functions responsible in the present invention, and may vary depending on the intention of the user or operator and practice, and embodiments below do not limit the right scope of the present invention, but are merely illustrative of the components presented in the claims of the present invention.

In the drawings, in order to describe clearly the invention, parts not related to the description are omitted, and identical or like reference signs denote identical or like components throughout the specification. Throughout the specification, when a part "includes" or "comprises" a component, it does not mean that the part excludes other component, but instead that the part may further include other components unless expressly stated to the contrary.

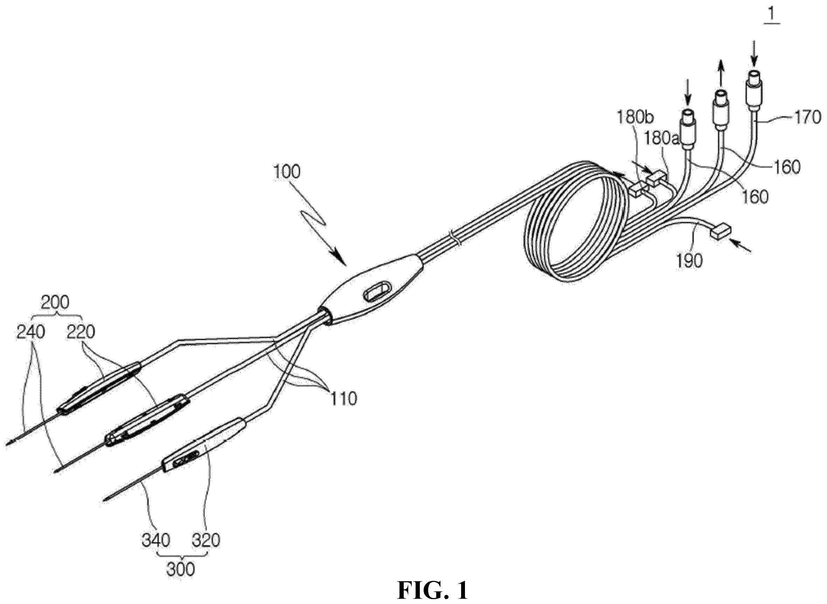

An electrode device 1 according to an embodiment of the present disclosure will be described with reference to FIGS. 1 to 5.

The electrode device 1 according to an embodiment of the present invention may generally include a main body 100, a plurality of high-frequency electrodes 200 for the cauterization of a lesion tissue, and a multi-functional electrode 300 for injecting a fluid or measuring the temperature of a surrounding tissue. In this embodiment, the electrode device 1 includes two high-frequency electrodes 200 and one multi-functional electrode 300, but is not limited thereto.

A high-frequency electrode cable 160, a multi-functional electrode cable 170, a pair of cooling tubes 180a and 180b, and a fluid tube 190 may be connected to the main body 100. Specifically, inside the high-frequency electrode cable 160, an electrode line 120 connected to a high-frequency generator (not shown) to supply a high frequency to the high-frequency electrode 200, and a temperature sensor line 150 connected to a temperature sensor 244 to be described later may be disposed, and in this embodiment, two high-frequency electrode cables 160 are provided corresponding to the two high-frequency electrodes 200, and one electrode line 120 and one temperature sensor line 150 are disposed within each of the cables 160. Additionally, cooling lines 130a and 130b may be disposed inside the pair of cooling tubes 180a and 180b, respectively so that they are connected to a cooling water pump (not shown) or the like to circulate and supply cooling water into each of the high-frequency electrodes 200. In this regard, the cooling line 130a may be disposed inside the cooling tube 180a as a line for injection of the cooling water, and the cooling line 130b may be disposed inside the cooling tube 180b as a line for recovery of the cooling water. In this embodiment, two cooling lines 130a are disposed inside the cooling tube 180a to supply the cooling water to the two high-frequency electrodes 200, respectively, and two cooling lines 130b are also disposed inside the cooling tube 180b to recover the cooling water from the two high-frequency electrodes 200, respectively.

Additionally, inside the fluid tube 190, a fluid line 140 may be disposed so that it may be connected to a syringe (not shown) or the like to supply fluid into the multi-functional electrode 300, and inside the multi-functional electrode cable 170, a temperature sensor line 150 may be disposed so that it may be connected to a temperature sensor 344 disposed inside the multi-functional electrode 300 as will be described later. In this way, the cables and the tubes may serve to protect the lines, and of course, may be omitted depending on an embodiment.

The electrode lines 120, the cooling lines 130a, 130b, the fluid line 140, and the temperature sensor lines 150 may reach the main body 100 through the cables and the tubes, and then penetrate the interior of the main body 100 to be distributed through flexible tubes 110 to the respective high-frequency electrodes 200 and the multi-functional electrode 300 as will be described later. Also, depending on an embodiment, cooling chambers in communication with the cooling lines 130a and 130b, respectively, or a fluid chamber in communication with the fluid line 140 may be formed inside the main body 100.

Each of the high-frequency electrodes 200 is connected with the main body 100 through the flexible tube 110, and specifically, includes a first gripping body 220 coupled to the main body 100 through the flexible tube 110, and a first electrode needle 240 coupled to the front portion of the first gripping body 220.

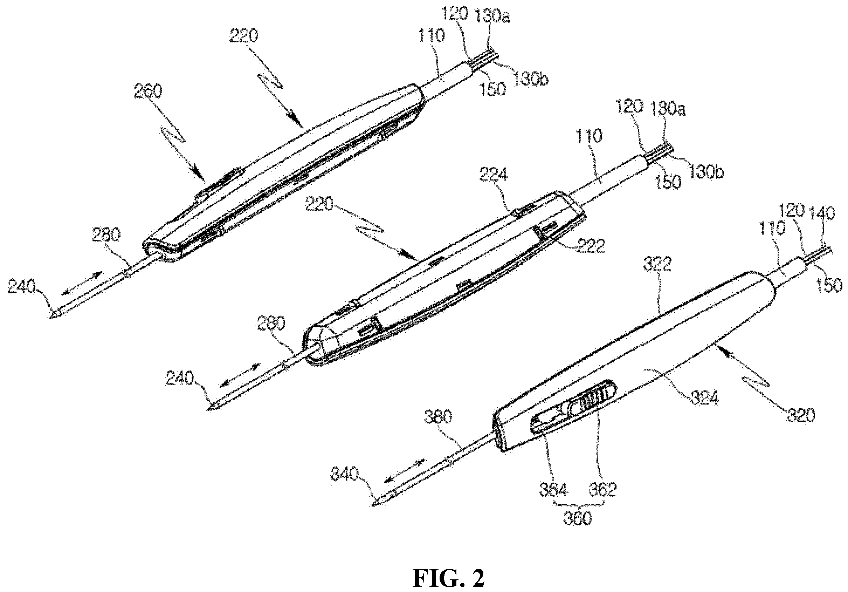

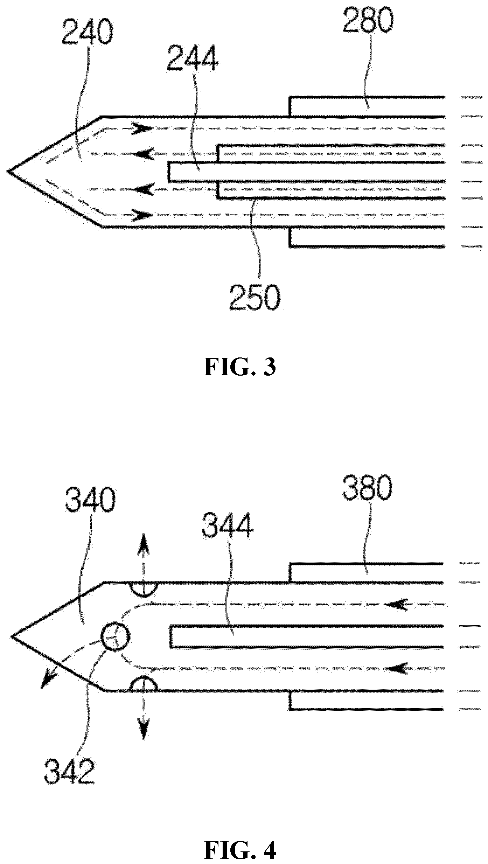

As shown in FIG. 2, the electrode line 120 and the cooling lines 130a and 130b may extend from the main body 100 through the inside of the flexible tube 110 to the first gripping body 220 to be distributed to the first electrode needle 240. In this embodiment, since the two high-frequency electrodes 200 are provided, the electrode lines 120 and the cooling lines 130a and 130b may be distributed to the respective high-frequency electrodes 200 as described above. Thereby, the high frequency can be supplied to the first electrode needle 240 through the electrode line 120, and the cooling water is supplied to the inside of the first electrode needle 240 through the cooling line 130a, and the cooling water which has been circulated inside the first electrode needle 240 can be recovered again through the cooling line 130b. For this purpose, as shown in FIG. 3, an inner pipe 250 may be provided inside the first electrode needle 240, so that the cooling water which has been supplied from the cooling line 130a into the inner pipe 250 can be supplied to the end portion of the first electrode needle 240, and the cooling water which has been circulated around the end portion of the first electrode needle 240 can be discharged along the space between the inner pipe 250 and the first electrode needle 240 again to be recovered through the cooling line 130b. Depending on an embodiment, a cooling chamber may be formed inside the first gripping body 220.

Moreover, inside the first electrode needle 240, the temperature sensor 244 may be provided for measuring the internal temperature of the first electrode needle 240 to prevent carbonization. As shown in FIG. 3, the temperature sensor 244 may be disposed at the end portion of the first electrode needle 240 to measure the internal temperature of the first electrode needle 240, which is subject to the circulation cooling by the cooling water. For this purpose, the temperature sensor line 150 connected to the temperature sensor 244 may be further provided, together with the electrode line 120, inside the high-frequency electrode cable 160. That is, in this embodiment, the two high-frequency electrode cables 160 are provided corresponding to the two high-frequency electrodes 200, so one temperature sensor line 150 may be disposed inside each of the cables 160 to be distributed to each of the high-frequency electrodes 200.

Also, the multi-functional electrodes 300 is connected with the main body 100 through the flexible tube 110, and specifically, includes a second gripping body 320 coupled to the main body 100 through the flexible tube 110, and a second electrode needle 340 coupled to the front portion of the second gripping body 320.

As shown in FIG. 2, the fluid line 140 and the temperature sensor line 150 may extend from the main body 100 through the inside of the flexible tube 110 to the second gripping body 320 to be distributed to the second electrode needle 340. As shown in FIG. 4, at the end portion of the second electrode needle 340, a plurality of injection holes 342 through which the fluid is injected are formed. Of course, depending on an embodiment, a single injection hole through which fluid is injected may be formed. Thereby, the fluid supplied into the second electrode needle 340 through the fluid line 140 may be injected to the outside of the electrode needle through the plurality of injection holes 342. For this purpose, it is natural that a fluid chamber may be formed inside the second gripping body 320 or that a fluid passage may be formed inside the second electrode needle 340.

Additionally, inside the second electrode needle 340, the temperature sensor 344 may be provided for measuring the temperature of a surrounding tissue 20. As shown in FIG. 4, the temperature sensor 344 is preferably disposed at the end portion of the second electrode needle 340 to be connected to the temperature sensor line 150 distributed to the second electrode needle 340. At this time, since the inside of the second electrode needle 340 is not cooled by the cooling water, it can measure the actual temperature of the surrounding tissue 20 by being brought into contact with the surrounding tissue 20. For this purpose, when the multi-functional electrode 300 measures the temperature of the surrounding tissue 20 with the temperature sensor 344, the fluid is not supplied through the fluid line 140.

Besides, when cauterization for hemostasis is necessary to eliminate bleeding that occurs when the multi-functional electrode 300 is removed from the insertion site, a high frequency may also be supplied to the second electrode needle 340. For this purpose, the electrode line 120 together with the temperature sensor line 150 may be provided inside the multi-functional electrode cable 170 so that it may be connected to the second electrode needle 340 to supply the high frequency thereto.

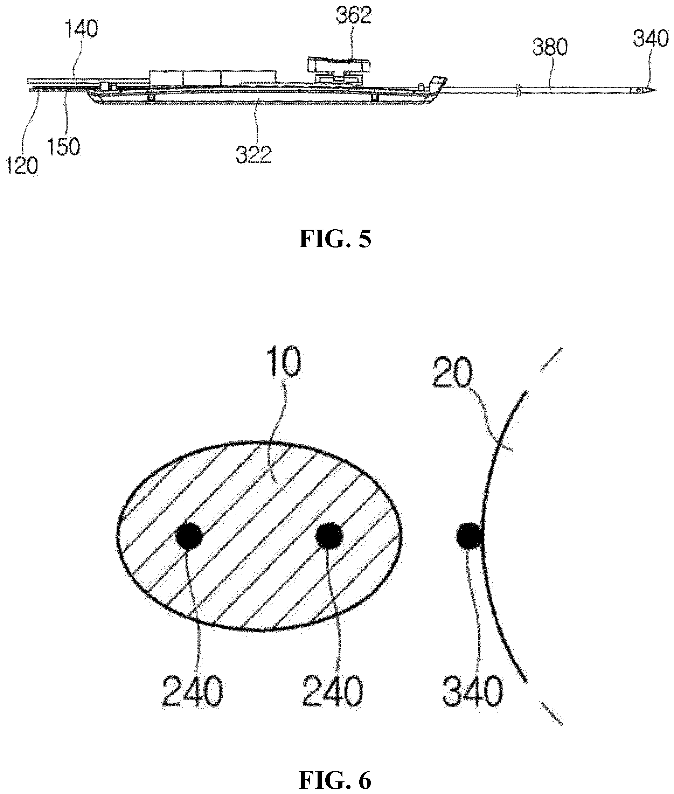

As shown in FIGS. 2 and 5, the multi-functional electrode 300 may further include a second movable part 360 movably installed on the second gripping body 320, and a second insulating tube 380 coupled to the second movable part 360 and arranged to surround the second electrode needle 340. When taking a closer look at the structure of the multi-functional electrode 300, the second gripping body 320 may be constituted with a base part 322 and a cover part 324 coupled to the base part 322. Additionally, the second movable part 360 may be constituted with a movement hole 364 and a movement button 362 which is movable within the movement hole 364.

FIG. 5 shows the multi-functional electrode with the flexible tube 110 and the cover part 324 of the second gripping body removed. As shown, the electrode line 120, the fluid line 140, and the temperature sensor line 150 may be distributed in the second gripping body 320 to the second electrode needle 340, and the movement button 362 of the second movable part 360 is movably installed on the base part 322 of the second gripping body. In addition, the movement hole 364 of the second movable part 360 is formed in the cover part 324, so that when the base part 322 of the second gripping body and the cover part 324 are combined to each other, the movement button 362 can be moved within this movement hole 364.

The second insulating tube 380 is fixed to the movement button 362 through bonding or the like, so that the second insulating tube 380 moves together with the movement button 362. Thereby, the user can easily adjust the position of the second insulating tube 380 by moving the movement button 362. In this regard, the second insulating tube 380 may be adjusted between a position where it covers the plurality of injection holes 342 and a position where it does not cover the plurality of injection holes 342.

Specifically, when the fluid is supplied to the multi-functional electrode 300 through the fluid line 140, the second insulating tube 380 may be adjusted so as not to cover the plurality of injection holes 342, and when the multi-functional electrode 300 measures the temperature with the temperature sensor 344, the second insulating tube 380 may be adjusted to cover the plurality of injection holes 342. Thereby, when the second electrode needle 340 of the multi-functional electrode contacts the surrounding tissue 20 to measure the temperature of the surrounding tissue 20, the second insulating tube 380 covers the plurality of injection holes 342, so that it is possible to prevent the tissue from entering the injection hole and blocking it.

As shown in FIG. 2, each of the high-frequency electrodes 200 may likewise further include a first movable part 260 movably installed on the first gripping body 220, and a first insulating tube 280 coupled to the first movable part 260 and arranged to surround the first electrode needle 240. Thereby, when the user moves the first movable part 260, the first insulating tube 280 can be moved as one unit, so the exposed length of the first electrode needle 240 of the high-frequency electrode can be easily adjusted depending on the size of the lesion tissue 10. Here, the first movable part 260, like the second movable part 360, may be constituted with a movement button and a movement hole. For example, the movement button may be formed to be movable in 5 mm units.

Depending on an embodiment, the first gripping body 220 of the high-frequency electrode and the second gripping body 320 of the multi-functional electrode may be integrally combined to or separated from each other. For this purpose, grooves and protrusions may be formed on each of the gripping bodies 220 and 320, and insertion coupling between the gripping bodies may be achieved. FIG. 2 shows grooves 222 and protrusions 224 formed on the first gripping body 220. Additionally, the outer diameter of the second electrode needle 340 of the multi-functional electrode may be smaller than the outer diameter of the first electrode needle 240 of the high-frequency electrode.

Hereinafter, a medical procedure using the above-described electrode device 1 will be briefly described. The user inserts the first electrode needle 240 so that it penetrates the lesion tissue 10, such as cancer tissue, while holding the first gripping body 220 of the high-frequency electrode, and then the user supplies the high frequency to the first electrode needle 240 through the electrode line 120 to cauterize and necrotize the lesion site 10 with high frequency heat. In this regard, as the position of the first insulating tube 280 is adjusted through the first movable part 260, the length of the exposed portion of the first electrode needle 240 which is not covered by the first insulating tube 280 can be adjusted.

Since each of the plurality of high-frequency electrodes 200 is coupled to the main body 100 through the flexible tube 110, the position of the high-frequency electrode can be freely adjusted, allowing the cauterization position to be easily adjusted. Additionally, depending on the area of the lesion tissue 10 and scattered locations of the lesion tissues, the first gripping bodies 220 of the plurality of high-frequency electrodes 200 can be combined to or separated from each other, so that the plurality of first electrode needles 240 can be used all at one time or individually.

At the same time, the fluid may be injected or the temperature of the surrounding tissue 20 may be measured through the multi-functional electrode 300. For example, if it is necessary to expand the cauterization range, saline solution may be injected through the fluid line 140, and the injected saline solution may be supplied into the second electrode needle 340 and injected to the outside (specifically, toward the first electrode needle of the high-frequency electrode) through the plurality of injection holes 342. At this time, the position of the second insulating tube 380 is adjusted so as not to cover the plurality of injection holes 342. Additionally, since the multi-functional electrode 300 is coupled to the main body 100 through the flexible tube 110, the position of the multi-functional electrode can be freely adjusted, allowing the fluid injection position to be easily adjusted. As the electrical conductivity of the first electrode needle 240 of the high-frequency electrode is improved by the saline solution injected in this way, the high frequency through the first electrode needle 240 acts to conduct electricity to a wider range, thereby expanding the cauterization range. Moreover, not only the size but also the shape of the cauterization can be adjusted through the fluid injection. Further, the carbonization phenomenon in which the lesion tissue 10 is scorched and sticks to the surface of the first electrode needle 240 of the high-frequency electrode by the saline solution can be prevented. Besides, if the patient's pain is high, a drug such as a local anesthetic or the like may be injected through the fluid line 140, and the pain can be alleviated by the drug injected through the plurality of injection holes 342.

For example, as shown in FIG. 6, when there are organs around the lesion tissue 10 to be treated, which should not be damaged, the second electrode needle 340 of the multi-functional electrode contacts the surrounding tissue 20 and measures the temperature of the surrounding tissue through the temperature sensor 344. At this time, the position of the second insulating tube 380 is adjusted so as to cover the plurality of injection holes 342. In particular, unlike the first electrode needle 240, the inside of the second electrode needle 340 is not subject to the circulation cooling by the cooling water, nor fluid is supplied through the fluid line 140 when measuring the temperature, so the actual temperature of the surrounding tissue 20 can be measured. By performing the medical procedure with reference to the actual temperature of the surrounding tissue 20 measured in this way, damage to the surrounding tissue 20 can be prevented.

The present invention is not limited to the above-described specific embodiments and descriptions, and various modifications may be made by anyone skilled in the art without departing from the technical idea of the present invention as claimed in the claims, and such modifications will fall within the scope of protection of the present invention.

### [INDUSTRIAL APPLICABILITY]

The present invention relates to an electrode device, and more specifically, to an electrode device including a high-frequency electrode for cauterization of a lesion tissue and a multi-functional electrode for fluid injection and temperature measurement of a surrounding tissue.

## Claims

1. An electrode device comprising:
a plurality of high-frequency electrodes for cauterization of a lesion tissue;
a multi-functional electrode for injecting a fluid or measuring a temperature of a surrounding tissue;
a temperature sensor provided inside the multi-functional electrode; and
a main body which has an electrode line for supplying a high frequency to each of the plurality of high-frequency electrodes, a cooling line for circulating and supplying cooling water to an inside of each of the plurality of high-frequency electrodes, a fluid line for supplying a fluid to an inside of the multi-functional electrode, and a temperature sensor line connected to the temperature sensor, and which is coupled to the plurality of high-frequency electrodes and the multi-functional electrode via flexible tubes, respectively.

2. The electrode device of claim 1, wherein when the multi-functional electrode measures the temperature of the surrounding tissue with the temperature sensor, no fluid is supplied through the fluid line.

3. The electrode device of claim 2, wherein each of the high-frequency electrodes includes a first gripping body coupled to the main body through a flexible tube; and a first electrode needle coupled to a front portion of the first gripping body, and
wherein the multi-functional electrode includes a second gripping body coupled to the main body through a flexible tube; and a second electrode needle coupled to a front portion of the second gripping body.

4. The electrode device of claim 3, wherein at an end portion of the second electrode needle, a plurality of injection holes through which a fluid is injected are formed.

5. The electrode device of claim 3, wherein the temperature sensor is disposed at an end portion of the second electrode needle.

6. The electrode device of claim 4, wherein the multi-functional electrode further includes:
a second movable part movably installed on the second gripping body; and
a second insulating tube coupled to the second movable part and arranged to surround the second electrode needle.

7. The electrode device of claim 6, wherein when the fluid is supplied to the multi-functional electrode through the fluid line, the second insulating tube does not cover the plurality of injection holes, and
wherein when the multi-functional electrode measures a temperature with the temperature sensor, the second insulating tube covers the plurality of injection holes.

8. The electrode device of claim 3, wherein each of the high-frequency electrodes further includes:
a first movable part movably installed on the first gripping body, and
a first insulating tube coupled to the first movable part and arranged to surround the first electrode needle.

9. The electrode device of claim 3, wherein the first gripping body and the second gripping body are integrally combined to or separable from each other.
